(19) Europäisches Patentamt
European Patent Office
Office européen des brevets

(11) Veröffentlichungsnummer: **0 022 495**
**B1**

(12) # EUROPÄISCHE PATENTSCHRIFT

(45) Veröffentlichungstag der Patentschrift: **16.06.82**

(51) Int. Cl.³: **C 07 D 471/04**, A 61 K 31/44 // (C07D471/04, 221/00, 235/00)

(21) Anmeldenummer: **80103613.8**

(22) Anmeldetag: **26.06.80**

(54) **Neue 2-Alkoxyphenyl-imidazo(4,5-b)pyridine, deren Herstellung, deren Verwendung zur Arzneimittelherstellung und die genannten Verbindungen enthaltende Arzneimittel.**

(30) Priorität: **11.07.79 DE 2927987**

(43) Veröffentlichungstag der Anmeldung: **21.01.81 Patentblatt 81/3**

(45) Bekanntmachung des Hinweises auf die Patenterteilung: **16.06.82 Patentblatt 82/24**

(84) Benannte Vertragsstaaten: **AT BE CH DE FR GB IT LI LU NL SE**

(56) Entgegenhaltungen:
**CH-A-373 392**
**DE-A-2 361 757**
**DE-A-2 604 748**

(73) Patentinhaber: **Dr. Karl Thomae GmbH, Postfach 720, D-7950 Biberach (Riss) (DE)**

(72) Erfinder: **Austel, Volkhard, Dr. Dipl.-Chem., Kapellenweg 7, D-7950 Biberach 1 (DE)**
Erfinder: **Reiffen, Manfred, Dr. Dipl.-Chem., Amriswilstrasse 7, D-7950 Biberach 1 (DE)**
Erfinder: **Heider, Joachim, Dr. Dipl.-Chem., Am Hang 3, D-7951 Warthausen 1 (DE)**
Erfinder: **Diederen, Willi, Dr., Haldenstrasse 1a, D-7950 Biberach 1 (DE)**

**EP 0 022 495 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# Neue 2-Alkoxyphenyl-imidazo[4,5-b]pyridine, deren Herstellung, deren Verwendung zur Arzneimittelherstellung und die genannten Verbindungen enthaltenden Arzneimittel

In der BE-PS 810545, die der DE-OS 2361757 entspricht (vergl. darin die Beispiele 120–186), werden u.a. bereits 2-Alkoxy-phenyl-imidazo[4,5-b]pyridine der allgemeinen Formel

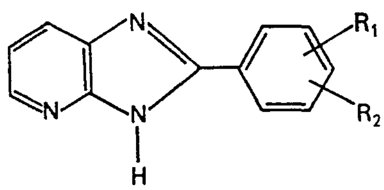

in der

$R_1$ eine Alkoxygruppe mit 1 bis 3 Kohlenstoffatomen und

$R_2$ eine durch eine Alkylsulfinylgruppe substituierte Alkoxygruppe mit 2 oder 3 Kohlenstoffatomen bedeuten, deren 3H Tautomere und physiologisch verträgliche Säureadditionssalze beschrieben. Diese weisen wertvolle pharmakologische Eigenschaften auf, insbesondere eine positiv-inotrope Wirkung.

Überraschenderweise wurde nun gefunden, dass die neuen 2-Alkoxyphenyl-imidazo[4,5-b]-pyridine der allgemeinen Formel

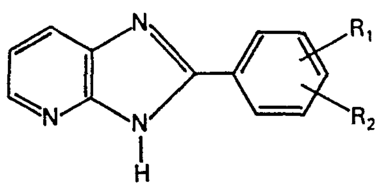

(I)

in der

$R_1$ eine Alkoxygruppe mit 1 bis 3 Kohlenstoffatomen und

$R_2$ eine durch eine Aralkylsulfinylgruppe mit 7 bis 9 Kohlenstoffatomen oder durch eine Phenylsulfinylgruppe substituierte Alkoxygruppe mit 2 oder 3 Kohlenstoffatomen darstellt, wobei der Phenylkern der Phenylsulfinylgruppe durch eine Nitrogruppe, durch Alkylgruppen mit 1 bis 3 Kohlenstoffatomen, Alkoxygruppen mit 1 bis 3 Kohlenstoffatomen und/oder Halogenatome mono-, di- oder trisubstituiert sein kann, gegenüber den aus der obengenannten Deutschen Offenlegungsschrift bekannten Verbindungen überlegene pharmakologische Eigenschaften aufweisen, insbesondere überlegene blutdrucksteigernde und/oder positiv inotrope Wirkungen.

Gegenstand der vorliegenden Erfindung sind somit die neuen 2-Alkoxyphenyl-imidazo[4,5-b]pyridine, deren 3H Tautomere, deren physiologisch verträgliche Säureadditionssalze mit anorganischen oder organischen Säuren und deren Verwendung als Arzneimittel sowie Verfahren zu ihrer Herstellung.

Für die bei der Definition der Reste $R_1$ und $R_2$ eingangs erwähnten Bedeutungen kommt somit für

$R_1$ insbesondere die Bedeutung der Methoxy-, Äthoxy-, Propoxy- oder Isopropoxygruppe und für

$R_2$ die der 2-Benzylsulfinyläthoxy-, 3-Benzylsulfinylpropoxy-, 2-Phenyläthylsulfinyläthoxy-, 3-Phenyläthylsulfinylpropoxy-, 2-Phenylpropylsulfinyläthoxy- oder 3-Phenylpropylsulfinylpropoxygruppe, die der Phenylsulfinyl-, Methylphenylsulfinyl-, Dimethylphenylsulfinyl-, Trimethylphenylsulfinyl-, Äthylphenylsulfinyl-, Isopropylphenylsulfinyl-, Methoxyphenylsulfinyl-, Dimethoxyphenylsulfinyl-, Trimethoxyphenylsulfinyl-, Diäthoxyphenylsulfinyl-, Propoxyphenylsulfinyl-, Nitrophenylsulfinyl-, Methyl-nitrophenylsulfinyl-, Methoxy-nitrophenylsulfinyl-, Fluorphenylsulfinyl-, Chlorphenylsulfinyl-, Bromphenylsulfinyl-, Difluorphenylsulfinyl-, Dichlorphenylsulfinyl-, Dibromphenylsulfinyl-, Fluor-chlorphenylsulfinyl-, Fluor-bromphenylsulfinyl-, Chlor-bromphenylsulfinyl-, Methyl-methoxyphenylsulfinyl-, Methyl-dimethoxyphenylsulfinyl-, Dimethyl-methoxyphenylsulfinyl-, Fluor-methoxyphenylsulfinyl-, Fluor-dimethoxyphenylsulfinyl-, Fluor-methylphenylsulfinyl-, Chlor-methoxyphenylsulfinyl-, Brom-dimethoxy-phenylsulfinyl- oder Fluormethylmethoxy-phenylsulfinyläthoxy- oder -propoxygruppe in Betracht.

Bevorzugte Verbindungen der allgemeinen Formel I sind jedoch diejenigen, in der

$R_1$ eine Methoxygruppe und

$R_2$ eine in 2-Stellung durch eine Benzylsulfinyl-, Phenylsulfinyl-, Chlorphenylsulfinyl-, Methylphenylsulfinyl-, Nitrophenylsulfinyl-, Methoxyphenylsulfinyl-, Dimethoxyphenylsulfinyl- oder dimethoxymethylphenylsulfinylgruppe substituierte Äthoxygruppe bedeuten. Besonders bevorzugte Verbindungen sind hierbei diejenigen, wenn der Rest $R_1$ in 4-Stellung und der Rest $R_2$ in 2-Stellung steht.

Erfindungsgemäss erhält man die neuen Verbindungen nach folgendem Verfahren:

Oxidation eines Imidazo[4,5-b]pyridins der allgemeinen Formel

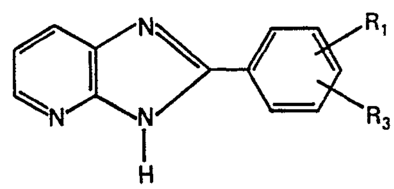

(II)

in der

$R_1$ wie eingangs definiert ist und

$R_3$ eine Alkoxygruppe mit 2 oder 3 Kohlenstoffatomen darstellt, welche durch eine Aralkylthiogruppe mit 7 bis 9 Kohlenstoffatomen oder durch eine gegebenenfalls durch eine Nitrogruppe, durch Alkylgruppen mit 1 bis 3 Kohlenstoffatomen, Alkoxygruppen mit 1 bis 3 Kohlenstoffatomen und/oder Halogenatome mono-, di- oder tri-

substituierte Phenylthiogruppe substituiert ist.

Die Oxidation wird vorzugsweise in einem Lösungsmittel, z.B. in Wasser, Wasser/Pyridin, Aceton, Eisessig, verdünnter Schwefelsäure oder Trifluoressigsäure, je nach dem verwendeten Oxidationsmittel zweckmässigerweise bei Temperaturen zwischen -80 und 100°C durchgeführt.

Vorzugsweise wird die Oxidation jedoch mit einem Äquivalent des verwendeten Oxidationsmittels durchgeführt, z.B. mit Wasserstoffperoxid in Eisessig bei 0 bis 20°C oder in Aceton bei 0 bis 60°C, mit einer Persäure wie Perameisensäure in Eisessig oder Trifluoressigsäure oder bei 0 bis 50°C, mit Natriummetaperjodat in wässrigem Methanol oder Äthanol bei 15 bis 25°C, mit tert.-Butylhypochlorit in Methanol bei -80 bis -30°C, mit Jodbenzoldichlorid in wässrigem Pyridin bei 0 bis 50°C, mit Salpetersäure in Eisessig bei 0 bis 20°C, mit Chromsäure in Eisessig oder in Aceton bei 0 bis 20°C und mit Sulfurylchlorid in Methylenchlorid bei -70°C, der hierbei erhaltene Thioäther-Chlor-Komplex wird zweckmässigerweise mit wässrigem Äthanol hydrolysiert.

Die erhaltenen Verbindungen der allgemeinen Formel I können anschliessend gewünschtenfalls in ihre physiologisch verträglichen Säureadditionssalze mit anorganischen oder organischen Säuren übergeführt werden. Als Säuren kommen hierfür beispielsweise Salzsäure, Bromwasserstoffsäure, Schwefelsäure, Phosphorsäure, Fumarsäure, Bernsteinsäure, Weinsäure, Zitronensäure, Milchsäure oder Maleinsäure in Betracht.

Die als Ausgangsstoffe verwendeten Verbindungen erhält man durch Umsetzung der entsprechenden Halogenalkoxy-imidazo[4,5-b]pyridine, welche in der BE-PS 810545 beschrieben werden, durch Umsetzung mit einem entsprechenden Mercaptan in Gegenwart einer Base wie Natriummethylat.

Wie bereits eingangs erwähnt, weisen die neuen Verbindungen der allgemeinen Formel I, deren 3H Tautomere und deren physiologisch verträgliche Säureadditionssalze überlegene pharmakologische Eigenschaften auf, insbesondere eine blutdrucksteigernde und/oder positiv-inotrope Wirkung.

Beispielsweise wurden die Verbindungen

A = 2-[2-(2-Phenylsulfinyl-ethoxy)-4-methoxyphenyl]-imidazo[4,5-b]pyridin,
B = 2-[2-(2-(4-Chlorphenylsulfinyl)-ethoxy)-4-methoxyphenyl]-imidazo[4,5-b]pyridin,
C = 2-[2-(2-(4-Methylphenylsulfinyl)-ethoxy)-4-methoxyphenyl]-imidazo[4,5-b]pyridin,
D = 2-[2-(2-(2-Methyl-4,5-dimethoxyphenylsulfinyl)-ethoxy)-4-methoxyphenyl]-imidazo[4,5-b]-pyridin und
E = 2-[2-(2-(4-Nitrophenylsulfinyl)-ethoxy)-4-methoxyphenyl]-imidazo[4,5-b]pyridin

auf ihre biologischen Eigenschaften wie folgt untersucht:

1. Bestimmung der Blutdruckwirkung und der positiv inotropen Wirkung an der narkotisierten Katze.

Die Untersuchungen wurden an Katzen durchgeführt, die mit Pentobarbital-Na (40 mg/kg i.p.) narkotisiert waren. Die Tiere atmeten spontan. Der arterielle Blutdruck wurde in der Aorta abdominalis mit einem Statham-Druckwandler (P 23 Dc) gemessen. Für die Erfassung der positiv inotropen Wirkung wurde mit einem Kathetertipmanometer (Millar PC-350 A) der Druck in der linken Herzkammer gemessen. Daraus wurde der Kontraktilitätsparameter $dp/dt_{max}$ mittels eines Analogdifferenzierers gewonnen.

Die zu untersuchenden Substanzen wurden in eine Vena femoralis injiziert. Als Lösungsmittel diente physiologische Kochsalz-Lösung oder Polydiol 200. Jede Substanz wurde an mindestens 3 Katzen geprüft, Dosis 2 mg/kg i.v.

Die nachfolgende Tabelle enthält die Mittelwerte:

| Substanz | Dosis mg/kg i.v. | Blutdruckänderung mm Hg | Zunahme dp/dt % |
|---|---|---|---|
| A | 2,0 | +33/20 | +115 |
| B | 2,0 | +60/42 | +101 |
| C | 2,0 | +70/45 | + 96 |
| D | 2,0 | +13/5 | + 45 |
| E | 2,0 | +67/32 | + 60 |

2. Akute Toxizität

Die akute Toxizität der zu untersuchenden Substanzen wurde an weissen Mäusen nach oraler Gabe einer einmaligen Dosis von 300 mg/kg orientierend bestimmt (Beobachtungszeit: 14 Tage):

| Substanz | $LD_{50}$ mg/kg p.o. |
|---|---|
| B | >300 (0 von 6 Tieren gestorben) |
| C | >300 (0 von 6 Tieren gestorben) |
| D | >300 (0 von 6 Tieren gestorben) |

Aufgrund ihrer pharmakologischen Eigenschaften eignen sich die erfindungsgemäss hergestellten Verbindungen der allgemeinen Formel I und deren 3H Tautomere sowie deren physiologisch verträgliche Säureadditionssalze mit anorganischen oder organischen Säuren zur Behandlung der chronischen Herzinsuffizienz und zur Behandlung des akuten Herzversagens im kardiogenen Schock.

Hierzu lassen sich die neuen Verbindungen, gegebenenfalls in Kombination mit anderen für diesen Zweck geeigneten Wirksubstanzen, in die üblichen pharmazeutischen Anwendungsformen wie Tabletten, Dragées, Pulver, Suppositorien, Suspensionen, Ampullen oder Tropfen einarbeiten. Die Einzeldosis am Erwachsenen beträgt hierbei 1-4× täglich 25-200 mg, vorzugsweise jedoch 50-100 mg.

Die nachfolgenden Beispiele sollen die Erfindung näher erläutern:

Beispiel 1

2-[2-(2-Phenylsulfinyl-ethoxy)-4-methoxyphenyl]-imidazo[4,5-b]pyridin

a) 2-[2-(2-Phenylthio-ethoxy)-4-methoxyphenyl]-imidazo[4,5-b]pyridin

Eine Lösung von 2,0 g (0,036 Mol) Natriummethylat in 50 ml absolutem Methanol wird mit 1,85 ml (0,018 Mol) Thiophenol und 4,6 g (0,015 Mol) 2-[2-(2-Chlorethoxy)-4-methoxyphenyl]-imidazo[4,5-b]pyridin-hydrochlorid versetzt. Nach 4stündigem Erhitzen unter Rückfluss wird im Vakuum eingeengt, in Wasser/Methylenchlorid gelöst, die organische Phase abgetrennt und über Magnesiumsulfat getrocknet. Eine Vorreinigung erfolgt über 50 g Kieselgel mit Methylenchlorid/Aceton (10/0 bis 80/20). Das Produkt wird ohne Nachreinigung weiter umgesetzt.
Ausbeute: 2,8 g (50% der Theorie).

b) 2-[2-(2-Phenylsulfinyl-ethoxy)-4-methoxyphenyl]-imidazo[4,5-b]pyridin

Eine Lösung von 2,8 g (0,0074 Mol) 2-[2-(2-Phenylthio-ethoxy)-4-methoxyphenyl]-imidazo[4,5-b]pyridin in 40 ml Eisessig wird mit 0,84 g Wasserstoffperoxid (30%ig) versetzt und 2 Stunden bei Raumtemperatur gerührt. Anschliessend wird mit 20 ml Wasser verdünnt, mit Kaliumkarbonat neutralisiert, dreimal mit je 50 ml Methylenchlorid extrahiert und über Magnesiumsulfat getrocknet. Die Reinigung erfolgt über 70 g Kieselgel mit Methylenchlorid/Aceton (10/0 bis 80/20).
Ausbeute: 2,1 g (72% der Theorie),
Schmelzpunkt: 185–186°C.

Beispiel 2

2-[2-(2-Benzylsulfinyl-ethoxy)-4-methoxyphenyl]-imidazo[4,5-b]pyridin

a) 2-[2-(2-Benzylthio-ethoxy)-4-methoxyphenyl]-imidazo[4,5-b]pyridin

Hergestellt analog Beispiel 1a aus 3,6 g 2-[2-(2-Chlorethoxy)-4-methoxyphenyl]-imidazo-[4,5-b]pyridin-hydrochlorid.
Ausbeute: 1,8 g (40% der Theorie).

b) 2-[2-(2-Benzylsulfinyl-ethoxy)-4-methoxyphenyl]-imidazo[4,5-b]pyridin

Hergestellt analog Beispiel 1b aus 1,8 g 2-[2-(2-Benzylthio-ethoxy)-4-methoxyphenyl]-imidazo[4,5-b]pyridin.
Ausbeute: 0,9 g (48% der Theorie),
Schmelzpunkt: 193–194°C.

Beispiel 3

2-[2-(2-(4-Chlorphenylsulfinyl)-ethoxy)-4-methoxyphenyl-imidazo[4,5-b]pyridin

a) 2-[2-(2-(4-Chlorophenylthio)-ethoxy)-4-methoxyphenyl]-imidazo[4,5-b]pyridin

Hergestellt analog Beispiel 1a aus 6,1 g 2-[2-(2-Chloroethoxy)-4-methoxyphenyl]-imidazo-[4,5-b]pyridin-hydrochlorid.
Ausbeute: 4,2 g (51% der Theorie).

b) 2-[2-(2-(4-Chlorophenylsulfinyl)-ethoxy)-4-methoxyphenyl]-imidazo[4,5-b]pyridin

Hergestellt analog Beispiel 1b aus 4,0 g 2-[2-(2-(4-Chlorophenylthio)-ethoxy)-4-methoxyphenyl]-imidazo[4,5-b]pyridin.
Ausbeute: 2,6 g (63% der Theorie),
Schmelzpunkt: 171–173°C.

Beispiel 4

2-[2-(2-(4-Methylphenylsulfinyl)-ethoxy)-4-methoxyphenyl]-imidazo[4,5-b]pyridin

a) 2-[2-(2-(4-methylphenylthio)-ethoxy)-4-methoxyphenyl]-imidazo[4,5-b]pyridin

Hergestellt analog Beispiel 1a aus 6,1 g 2-[2-(2-Chloroethoxy)-4-methoxyphenyl]-imidazo-[4,5-b]pyridin-hydrochlorid.
Ausbeute: 1,4 g (18% der Theorie).

b) 2-[2-(2-(4-Methylphenylsulfinyl)-ethoxy)-4-methoxyphenyl]-imidazo[4,5-b]pyridin

Hergestellt analog Beispiel 1b aus 1,3 g 2-[2-(2-(4-Methylphenylthio)-ethoxy)-4-methoxyphenyl]-imidazo[4,5-b]pyridin.
Ausbeute: 1,0 g (77% der Theorie),
Schmelzpunkt: 76–120°C.

Beispiel 5

2-[2-(2-(4-Methoxyphenylsulfinyl)-ethoxy)-4-methoxyphenyl]-imidazo[4,5-b]pyridin

a) 2-[2-(2-(4-Methoxyphenylthio)-ethoxy)-4-methoxyphenyl]-imidazo[4,5-b]pyridin

Hergestellt analog Beispiel 1a aus 4,6 g 2-[2-(2-Chloroethoxy)-4-methoxyphenyl]-imidazo-[4,5-b]pyridin-hydrochlorid.
Ausbeute: 4,9 g (80% der Theorie).

b) 2-[2-(2-(4-Methoxyphenylsulfinyl)-ethoxy)-4-methoxyphenyl]-imidazo[4,5-b]pyridin

Hergestellt analog Beispiel 1b aus 4,8 g 2-[2-(2-(4-Methoxyphenylthio)-ethoxy)-4-methoxyphenyl]-imidazo[4,5-b]pyridin.
Ausbeute: 1,6 g (32% der Theorie),
Schmelzpunkt: 198–201°C.

Beispiel 6

2-[2-(2-(2-Methoxyphenylsulfinyl)-ethoxy)-4-methoxyphenyl]-imidazo[4,5-b]pyridin

a) 2-[2-(2-(2-Methoxyphenylthio)-ethoxy)-4-methoxyphenyl]-imidazo[4,5-b]pyridin

Hergestellt analog Beispiel 1a aus 4,6 g 2-[2-(2-Chloroethoxy)-4-methoxyphenyl]-imidazo-[4,5-b]pyridin-hydrochlorid.
Ausbeute: 2,3 g (38% der Theorie).

b) 2-[2-(2-(2-Methoxyphenylsulfinyl)-ethoxy)-4-methoxyphenyl]-imidazo[4,5-b]pyridin

Hergestellt analog Beispiel 1b aus 2,3 g 2-[2-(2-(2-Methoxyphenylthio)-ethoxy)-4-methoxyphenyl]-imidazo[4,5-b]pyridin.

Ausbeute: 1,3 g (54% der Theorie), Schmelzpunkt: 128–132°C.

Beispiel 7

2-[2-(2-(3,4-Dimethoxyphenylsulfinyl)-ethoxy)-4-methoxyphenyl]-imidazo[4,5-b]pyridin

a) 2-[2-(2-(3,4-Dimethoxyphenylthio)-ethoxy)-4-methoxyphenyl]-imidazo[4,5-b]pyridin

Hergestellt analog Beispiel 1a aus 4,6 g 2-[2-(2-Chloroethoxy)-4-methoxyphenyl]-imidazo-[4,5-b]pyridin-hydrochlorid.
Ausbeute: 4,3 g (65% der Theorie).

b) 2-[2-(2-(3,4-Dimethoxyphenylsulfinyl)-ethoxy)-4-methoxyphenyl]-imidazo[4,5-b]pyridin

Hergestellt analog Beispiel 1 aus 4,2 g 2-[2-(2-(3,4-Dimethoxyphenylthio)-ethoxy)-4-methoxyphenyl]-imidazo[4,5-b]pyridin.
Ausbeute: 2,7 g (62% der Theorie), Schmelzpunkt: 155–159°C.

Beispiel 8

2-[2-(2-(2-Methyl-4,5-dimethoxyphenylsulfinyl)-ethoxy)-4-methoxyphenyl]-imidazo[4,5-b]-pyridin

a) 2-[2-(2-(2-Methyl-4,5-dimethoxyphenylthio)-ethoxy)-4-methoxyphenyl]-imidazo-[4,5-b]pyridin

Hergestellt analog Beispiel 1a aus 4,6 g 2-[2-(2-Chloroethoxy)-4-methoxyphenyl]-imidazo-[4,5-b]pyridin-hydrochlorid.
Ausbeute: 3,8 g (56% der Theorie).

b) 2-[2-(2-(2-Methyl-4,5-dimethoxyphenylsulfinyl)-ethoxy)-4-methoxyphenyl]-imidazo[4,5-b]-pyridin

Hergestellt analog Beispiel 1b aus 3,7 g 2-[2-(2-(2-Methyl-4,5-dimethoxyphenylthio)-ethoxy)-4-methoxyphenyl]-imidazo[4,5-b]pyridin.
Ausbeute: 1,9 g (51% der Theorie), Schmelzpunkt: 170–172°C.

Beispiel 9

2-[2-(2-(4-Nitrophenylsulfinyl)-ethoxy)-4-methoxyphenyl]-imidazo[4,5-b]pyridin

a) 2-[2-(2-(4-Nitrophenylthio)-ethoxy)-4-methoxyphenyl]-imidazo[4,5-b]pyridin

Hergestellt analog Beispiel 1a aus 4,6 g 2-[2-(2-Chloroethoxy)-4-methoxyphenyl-imidazo-[4,5-b]pyridin-hydrochlorid.
Ausbeute: 3,8 g (60% der Theorie).

b) 2-[2-(2-(4-Nitrophenylsulfinyl)-ethoxy)-4-methoxyphenyl)-imidazo[4,5-b]pyridin

Hergestellt analog Beispiel 1b aus 4,7 g 2-[2-(2-(4-Nitrophenylthio)-ethoxy)-4-methoxyphenyl]-imidazo[4,5-b]pyridin.
Ausbeute: 0,94 g (19% der Theorie), Schmelzpunkt: 202,5–204,5°C.

Beispiel 10

2-[2-(2-(2,4-Dimethoxyphenylsulfinyl)-ethoxy)-4-methoxyphenyl]-imidazo[4,5-b]pyridin

a) 2-[2-(2-(2,4-Dimethoxyphenylthio)-ethoxy)-4-methoxyphenyl]-imidazo[4,5-b]pyridin

Hergestellt analog Beispiel 1a aus 4,0 g 2-[2-(2-Chloroethoxy)-4-methoxyphenyl]-imidazo-[4,5-b]pyridin-hydrochlorid.
Ausbeute: 4,6 g (70% der Theorie).

b) 2-[2-(2-(2,4-Dimethoxyphenylsulfinyl)-ethoxy)-4-methoxyphenyl]-imidazo[4,5-b]pyridin

Hergestellt analog Beispiel 1b aus 2,3 g 2-[2-(2-(2,4-Dimethoxyphenylthio)-ethoxy)-4-methoxyphenyl-imidazo[4,5-b]pyridin.
Ausbeute: 1,8 g (78% der Theorie), Schmelzpunkt: 176–179°C.

Beispiel A

Tabletten zu 100 mg 2-[2-(2-Phenylsulfinylethoxy)-4-methoxyphenyl]-imidazo[4,5-b]pyridin

Zusammensetzung:

| 1 Tablette enthält: | |
|---|---|
| Wirksubstanz | 100,0 mg |
| Milchzucker | 50,0 mg |
| Polyvinylpyrrolidon | 5,0 mg |
| Carboxymethylcellulose | 19,0 mg |
| Magnesiumstearat | 1,0 mg |
| | 175,0 mg |

| | |
|---|---|
| Feuchtsiebung: | 1,5 mm |
| Trocknen: | Umlufttrockenschrank 50°C |
| Trockensieben: | 1 mm |

Dem Granulat die restlichen Hilfsstoffe zumischen und Endmischung zu Tabletten verpressen.

| | |
|---|---|
| Tablettengewicht: | 175 mg |
| Stempel: | 8 mm∅ |

Beispiel B

Dragées zu 50 mg 2-[2-(2-Phenylsulfinyl-ethoxy)-4-methoxyphenyl]-imidazo[4,5-b]pyridin

1 Dragéekern enthält:

| | |
|---|---|
| Wirksubstanz | 50,0 mg |
| Maisstärke getr. | 20,0 mg |
| Lösliche Stärke | 2,0 mg |
| Carboxymethylcellulose | 7,0 mg |
| Magnesiumstearat | 1,0 mg |
| | 80,0 mg |

Wirkstoff und Stärke mit wässriger Lösung der löslichen Stärke gleichmässig befeuchten.

| | |
|---|---|
| Feuchtsiebung: | 1,0 mm |
| Trockensiebung: | 1,0 mm |
| Trocknung: | 50°C im Umlufttrockenschrank |

Granulat und restliche Hilfsstoffe mischen und zu Kernen verpressen.

| | |
|---|---|
| Kerngewicht: | 80 mg |

| | |
|---|---|
| Stempel: | 6 mm |
| Wölbungsradius: | 5 mm |

Die fertigen Kerne werden auf übliche Weise mit einem Zuckerüberzug im Dragierkessel versehen.

| | |
|---|---|
| Dragéegewicht: | 120 mg |

Beispiel C

Suppositorien zu 75 mg 2-[2-(2-Phenylsulfinylethoxy)-4-methoxyphenyl]-imidazo[4,5-b]pyridin

1 Zäpfchen enthält:

| | |
|---|---|
| Wirksubstanz | 75,0 mg |
| Zäpfchenmasse (z.B. Witepsol H 19 und Witepsol W 45) | 1625,0 mg |
| | 1700,0 mg |

Herstellungsverfahren:

Die Zäpfchenmasse wird geschmolzen. Bei 38°C wird die gemahlene Wirksubstanz in der Schmelze homogen dispergiert. Es wird auf 35°C abgekühlt und in vorgekühlte Suppositorienformen ausgegossen.

| | |
|---|---|
| Zäpfchengewicht: | 1,7 g |

Beispiel D

Ampullen zu 50 mg 2-[2-(2-Phenylsulfinyl-ethoxy)-4-methoxyphenyl]-imidazo[4,5-b]pyridin

1 Ampulle enthält:

| | | |
|---|---|---|
| Wirksubstanz | | 50,0 mg |
| Sorbit | | 250,0 mg |
| Dest. Wasser | ad | 5,0 ml |

Herstellungsverfahren:

Die Wirksubstanz und Sorbit werden in dest. Wasser gelöst, dann wird auf das angegebene Volumen aufgefüllt und steril abfiltriert.

| | |
|---|---|
| Abfüllung: | in Ampullen zu 5 ml |
| Sterilisation: | 20 Minuten bei 120°C |

Beispiel E

Tropfen zu 250 mg pro 5 ml 2-[2-(2-Phenylsulfinyl-ethoxy)-4-methoxyphenyl]-imidazo[4,5-b]pyridin

| | | |
|---|---|---|
| Wirksubstanz | | 5,0 g |
| p-Oxybenzoesäuremethylester | | 0,035 g |
| p-Oxybenzoesäurepropylester | | 0,015 g |
| Anisöl | | 0,05 g |
| Menthol | | 0,06 g |
| Saccharin-Natrium | | 1,0 g |
| Glycerin | | 10,0 g |
| Äthanol | | 40,0 g |
| Dest. Wasser | ad | 100,0 ml |

Herstellungsverfahren:

Die Benzoesäureester werden in Äthanol gelöst und anschliessend das Anisöl und das Menthol zugegeben. Dann wird die Wirksubstanz, Glycerin und Saccharin-Natrium im Wasser gelöst zugegeben. Die Lösung wird anschliessend klar filtriert.

**Patentansprüche**

für die Vertragsstaaten BE, CH/LI, DE, FR, GB, IT, LU, NL, SE

1. 2-Alkoxyphenyl-imidazo[4,5-b]pyridine der allgemeinen Formel

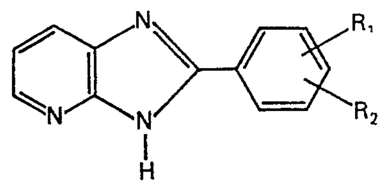

(I)

in der

$R_1$ eine Alkoxygruppe mit 1 bis 3 Kohlenstoffatomen und

$R_2$ eine durch eine Aralkylsulfinylgruppe mit 7 bis 9 Kohlenstoffatomen oder durch eine Phenylsulfinylgruppe substituierte Alkoxygruppe mit 2 oder 3 Kohlenstoffatomen darstellt, wobei der Phenylkern der Phenylsulfinylgruppen durch eine Nitrogruppe, durch Alkylgruppen mit 1 bis 3 Kohlenstoffatomen, Alkoxygruppen mit 1 bis 3 Kohlenstoffatomen und/oder Halogenatome mono-, di- oder trisubstituiert sein kann, deren 3H Tautomere und deren physiologisch verträgliche Säureadditionssalze mit anorganischen oder organischen Säuren.

2. Verbindungen der allgemeinen Formel I gemäss Anspruch 1, in der

$R_2$ die Methoxygruppe und

$R_2$ eine in 2-Stellung durch eine Benzylsulfinyl-, Phenylsulfinyl-, Chlorphenylsulfinyl-, Methylphenylsulfinyl-, Nitrophenylsulfinyl-, Methoxyphenylsulfinyl-, Dimethoxyphenylsulfinyl-, oder Dimethoxy-methylphenylsulfinylgruppe substituierte Äthoxygruppe bedeuten, deren 3H Tautomere und deren physiologisch verträgliche Säureadditionssalze mit anorganischen oder organischen Säuren.

3. Verbindungen der allgemeinen Formel I gemäss Anspruch 1, in der

$R_1$ und $R_2$ wie im Anspruch 2 definiert sind, $R_1$ in 4-Stellung und $R_2$ in 2-Stellung des Phenylkerns steht, deren 3H-Tautomeren und deren physiologisch verträgliche Säureadditionssalze mit anorganischen oder organischen Säuren.

4. 2-[2-(2-Phenylsulfinyl-ethoxy]-4-methoxyphenyl]-imidazo[4,5-b]pyridin und dessen Säureadditionssalze.

5. 2-[2-(2-(4-Chlorphenylsulfinyl)-ethoxy)-4-methoxyphenyl]-imidazo[4,5-b]pyridin und dessen Säureadditionssalze.

6. 2-[2-(2-(4-Methylphenylsulfinyl)-ethoxy)-4-methoxyphenyl]-imidazo[4,5-b]pyridin und dessen Säureadditionssalze.

7. 2-[2-(2-(2-Methyl-4,5-dimethoxyphenylsulfinyl)-ethoxy)-4-methoxyphenyl]-imidazo[4,5-b]pyridin und dessen Säureadditionssalze.

8. Verwendung der neuen Verbindungen gemäss den Ansprüchen 1-7 zur Herstellung eines Arzneimittels mit blutdrucksteigender und/oder positiv inotropen Wirkung auf nichtchemischem Wege.

9. Arzneimittel, enthaltend eine Verbindung gemäss den Ansprüchen 1-7 neben einem oder mehreren inerten Trägerstoffen und/oder Verdünnungsmitteln.

10. Verfahren zur Herstellung von neuen 2-Alkoxyphenyl-imidazo[4,5-b]pyridinen der allgemeinen Formel gemäss Anspruch 1

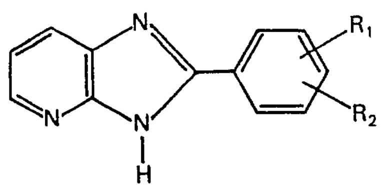

(I)

in der

$R_1$ eine Alkoxygruppe mit 1 bis 3 Kohlenstoffatomen und

$R_2$ eine durch eine Aralkylsulfinylgruppe mit 7 bis 9 Kohlenstoffatomen oder eine Phenylsulfinylgruppe substituierte Alkoxygruppe mit 2 oder 3 Kohlenstoffatomen darstellen, wobei der Phenylkern der Phenylsulfinylgruppe durch eine Nitrogruppe, durch Alkylgruppen mit 1 bis 3 Kohlenstoffatomen, Alkoxygruppen mit 1 bis 3 Kohlenstoffatomen und/oder Halogenatome mono-, di- oder trisubstituiert sein kann, sowie von deren 3H Tautomeren und deren physiologisch verträglichen Säureadditionssalzen mit anorganischen oder organischen Säuren, dadurch gekennzeichnet, dass ein Imidazo[4,5-b]pyridin der allgemeinen Formel

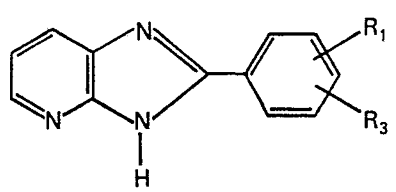

(II)

in der

$R_1$ wie eingangs definiert ist und

$R_3$ eine Alkoxygruppe mit 2 oder 3 Kohlenstoffatomen darstellt, welche durch eine Aralkylthiogruppe mit 7 bis 9 Kohlenstoffatomen oder durch eine gegebenenfalls durch eine Nitrogruppe, durch Alkylgruppen mit 1 bis 3 Kohlenstoffatomen, Alkoxygruppen mit 1 bis 3 Kohlenstoffatomen und/oder Halogenatome mono-, di- oder trisubstituierte Phenylthiogruppe substituiert ist, oxidiert wird und gewünschtenfalls anschliessend eine erhaltene Verbindung der allgemeinen Formel I in ihre physiologisch verträgliche Säureadditionssalze mit anorganischen oder organischen Säuren übergeführt wird.

## Patentansprüche

für den Vertragsstaat: AT

1. Verfahren zur Herstellung von neuen 2-Alkoxyphenyl-imidazo[4,5-b]pyridinen der allgemeinen Formel

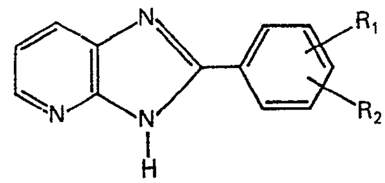

(I)

in der

$R_1$ eine Alkoxygruppe mit 1 bis 3 Kohlenstoffatomen und

$R_2$ eine durch eine Aralkylsulfinylgruppe mit 7 bis 9 Kohlenstoffatomen oder eine Phenylsulfinylgruppe substituierte Alkoxygruppe mit 2 oder 3 Kohlenstoffatomen darstellen, wobei der Phenylkern der Phenylsulfinylgruppe durch eine Nitrogruppe, durch Alkylgruppen mit 1 bis 3 Kohlenstoffatomen, Alkoxygruppen mit 1 bis 3 Kohlenstoffatomen und/oder Halogenatome mono-, di- oder trisubstituiert sein kann, sowie von deren 3H Tautomeren und deren physiologisch verträglichen Säureadditionssalzen mit anorganischen oder organischen Säuren, dadurch gekennzeichnet, dass ein Imidazo[4,5-b]pyridin der allgemeinen Formel

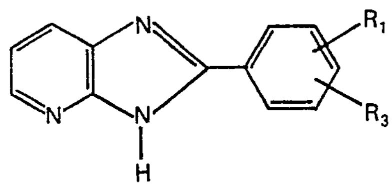

(II)

in der

$R_1$ wie eingangs definiert ist und

$R_3$ eine Alkoxygruppe mit 2 oder 3 Kohlenstoffatomen darstellt, welche durch eine Aralkylthiogruppe mit 7 bis 9 Kohlenstoffatomen oder durch eine gegebenenfalls durch eine Nitrogruppe, durch Alkylgruppen mit 1 bis 3 Kohlenstoffatomen, Alkoxygruppen mit 1 bis 3 Kohlenstoffatomen und/oder Halogenatome mono-, di- oder trisubstituierte Phenylthiogruppe substituiert ist, oxidiert wird und gewünschtenfalls anschliessend eine erhaltene Verbindung der allgemeinen Formel I in ihre physiologisch verträgliche Säureadditionssalze mit anorganischen oder organischen Säuren übergeführt wird.

2. Verfahren gemäss Anspruch 1 zur Herstellung von neuen 2-Alkoxyphenyl-imidazo[4,5-b]pyridinen der allgemeinen Formel

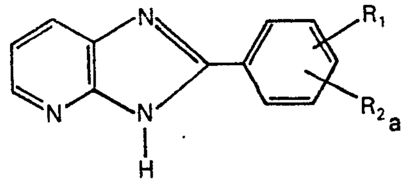

(Ia)

in der

$R_1$ wie im Anspruch 1 definiert ist und

$R_{2a}$ eine durch eine Aralkylsulfinylgruppe mit 7 bis 9 Kohlenstoffatomen oder eine Phenylsulfinylgruppe substituierte Alkoxygruppe mit 2 oder 3 Kohlenstoffatomen darstellen, wobei der Phenylkern der Phenylsulfinylgruppe durch Alkylgruppen mit 1 bis 3 Kohlenstoffatomen, Alkoxygruppen mit 1 bis 3 Kohlenstoffatomen und/oder Halogenatome mono-, di- oder trisubstituiert sein kann, sowie von deren 3H Tautomeren und deren physiologisch verträglichen Säureadditionssalzen mit anorganischen oder organischen Säuren, dadurch gekennzeichnet, dass ein Imidazo[4,5-b]pyridin der allgemeinen Formel

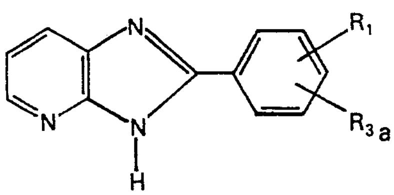

(IIa)

in der

$R_1$ wie im Anspruch 1 definiert ist und

$R_{3a}$ eine Alkoxygruppe mit 2 oder 3 Kohlenstoffatomen darstellt, welche durch eine Aralkylthiogruppe mit 7 bis 9 Kohlenstoffatomen oder durch eine gegebenenfalls durch Alkylgruppen mit 1 bis 3 Kohlenstoffatomen, Alkoxygruppen mit 1 bis 3 Kohlenstoffatomen und/oder Halogenatome mono-, di- oder trisubstituierte Phenylthiogruppe substituiert ist, oxidiert wird und gewünschtenfalls anschliessend eine erhaltene Verbindung der allgemeinen Formel I in ihre physiologisch verträgliche Säureadditionssalze mit anorganischen oder organischen Säuren übergeführt wird.

Priorität: 11.07.1979 (P 29 27 987.6)

3. Verfahren gemäss den Ansprüchen 1 und 2, dadurch gekennzeichnet, dass die Umsetzung in einem Lösungsmittel bei Temperaturen zwischen -80 und 100°C durchgeführt wird.

4. Verfahren gemäss den Ansprüchen 1 bis 3, dadurch gekennzeichnet, dass die Oxidation mit einem Äquivalent des verwendeten Oxidationsmittels und bei Raumtemperatur durchgeführt wird.

5. Verfahren gemäss den Ansprüchen 1 bis 4, dadurch gekennzeichent, dass als Oxidationsmittel Wasserstoffperoxid verwendet wird.

**Claims**

for the contracting states BE, CH, LI, DE, FR, GB, IT, LU, NL, SE

1. 2-Alkoxyphenyl-imidazo[4,5-b]pyridines of general formula

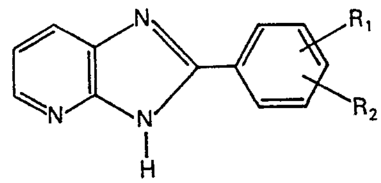

(I)

wherein

$R_1$ represents an alkoxy group with 1 to 3 carbon atoms and

$R_2$ represents an alkoxy group with 2 or 3 carbon atoms substituted by an aralkylsulfinyl group with 7 to 9 carbon atoms or by a phenylsulfinyl group, whilst the phenyl nucleus of the phenylsulfinyl groups may be mono-, di- or trisubstituted by a nitro group, by alkyl groups with 1 to 3 carbon atoms, by alkoxy groups with 1 to 3 carbon atoms and/or by halogen atoms, and the 3H tautomers thereof and the physiologically acceptable acid addition salts thereof with inorganic or organic acids.

2. Compounds of general formula I as claimed in claim 1, wherein

$R_1$ represents the methoxy group and

$R_2$ represents an ethoxy group substituted in the 2 position by a benzylsulfinyl, phenylsulfinyl, chlorophenylsulfinyl, methylphenylsulfinyl, nitrophenylsulfinyl, methoxyphenylsulfinyl, dimethoxyphenylsulfinyl or dimethoxymethylphenylsulfinyl group, and the 3H tautomers thereof and the physiologically acceptable acid addition salts thereof with inorganic or organic acids.

3. Compounds of general formula I as claimed in claim 1 wherein

$R_1$ and $R_2$ are defined as in claim 2, $R_1$, is in the 4-position and $R_2$ is in the 2-position of the phenyl nucleus and the 3H tautomers thereof and the physiologically acceptable acid salts thereof with inorganic or organic acids.

4. 2-[2-(2-Phenylsulfinyl-ethoxy)-4-methoxyphenyl]-imidazo[4,5-b]pyridine and the acid addition salts thereof.

5. 2-[2-(2-(4-Chlorophenylsulfinyl)-ethoxy)-4-methoxyphenyl]-imidazo[4,5-b]pyridine and the acid addition salts thereof.

6. 2-[2-(2-(4-Methylphenylsulfinyl)-ethoxy)-4-methoxyphenyl]-imidazo[4,5-b]pyridine and the acid addition salts thereof.

7. 2-[2-(2-Methyl-4,5-dimethoxyphenylsulfinyl)-ethoxy)-4-methoxyphenyl]-imidazo[4,5-b]-pyridine and the acid addition salts thereof.

8. Use of the new compounds as claimed in claims 1 to 7 for the preparation of a pharmaceutical composition with a hypertensive and/or positive inotropic effect, by a non-chemical method.

9. Pharmaceutical compositions, containing a compound as claimed in claims 1 to 7, together with one or more inert carriers and/or diluents.

10. Process for the preparation of new 2-alkoxyphenyl-imidazo-[4,5-b]pyridines of general formula:

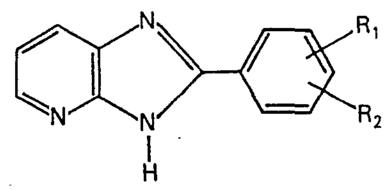

(I)

as claimed in claim 1,
wherein

$R_1$ represents an alkoxy group with 1 to 3 carbon atoms and

$R_2$ represents an alkoxy group with 2 or 3 carbon atoms substituted by an aralkylsulfinyl group with 7 to 9 carbon atoms or by a phenylsulfinyl group, whilst the phenyl nucleus of the phenylsulfinyl group may be mono-, di- or tri-substituted by a nitro group, by alkyl groups with 1 to 3 carbon atoms, by alkoxy groups with 1 to 3 carbon atoms and/or by halogen atoms, and the 3H tautomers thereof and the physiologically acceptable acid addition salts thereof with inorganic or organic acids, characterised in that an imidazo[4,5-b]pyridine of general formula

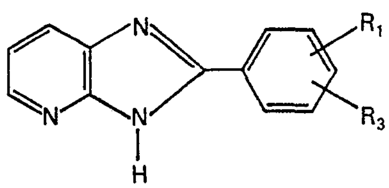

(II)

wherein

$R_1$ is as hereinbefore defined and

$R_3$ represents an alkoxy group with 2 or 3 carbon atoms which is substituted by an aralkylthio group with 7 to 9 carbon atoms or by a phenylthio group optionally mono-, di- or tri-substituted by a nitro group, by alkyl groups with 1 to 3 carbon atoms, by alkoxy groups with 1 to 3 carbon atoms and/or by halogen atoms, is oxidised and subsequently, if desired, a compound of general formula I thus obtained is converted into the physiologically acceptable acid addition salts thereof with inorganic or organic acids.

**Claims**

for the contracting states AT

1. Process for the preparation of new 2-alkoxyphenyl-imidazo-[4,5-b]pyridines of general formula:

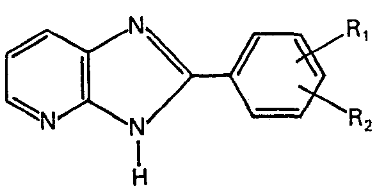

(I)

wherein

$R_1$ represents an alkoxy group with 1 to 3 carbon atoms and

$R_2$ represents an alkoxy group with 2 or 3 carbon atoms substituted by an aralkylsulfinyl group with 7 to 9 carbon atoms or by a phenylsulfinyl group, whilst the phenyl nucleus of the phenylsulfinyl group may be mono-, di- or tri-substituted by a nitro group, by alkyl groups with 1 to 3 carbon atoms, by alkoxy groups with 1 to 3 carbon atoms and/or by halogen atoms, and the 3H tautomers thereof and the physiologically acceptable acid addition salts thereof with inorganic or organic acids, characterised in that an imidazo[4,5-b]pyridine of general formula

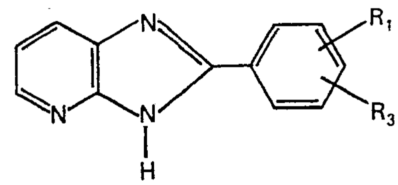

(II)

wherein

$R_1$ is as hereinbefore defined and

$R_3$ represents an alkoxy group with 2 to 3 carbon atoms which is substituted by an aralkylthio group with 7 to 9 carbon atoms or by a phenylthio group optionally mono-, di- or tri-substituted by a nitro group, by alkyl groups with 1 to 3 carbon atoms, by alkoxy groups with 1 to 3 carbon atoms and/or by halogen atoms, is oxidised and subsequently, if desired, a compound of general formula I thus obtained is converted into the physiologically acceptable acid addition salts thereof with inorganic or organic acids.

2. A process as claimed in claim 1 for the preparation of new 2-alkoxyphenyl-imidazo[4,5-b]pyridines of general formula

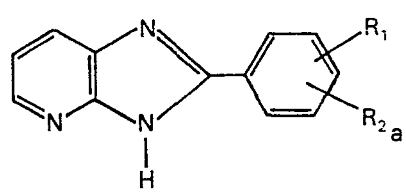

(Ia)

wherein

$R_1$ is defined as in claim 1 and

$R_{2a}$ represents an alkoxy group with 2 or 3 carbon atoms substituted by an aralkylsulfinyl group with 7 to 9 carbon atoms or by a phenylsulfinyl group, whilst the phenyl nucleus of the phenylsulfinyl group may be mono-, di- or trisubstituted by alkyl groups with 1 to 3 carbon atoms, alkoxy groups with 1 to 3 carbon atoms and/or halogen atoms, and the 3H tautomers thereof and the physiologically acceptable acid addition salts thereof with organic or inorganic acids, characterised in that an imidazo-[4,5-b]pyridine of general formula

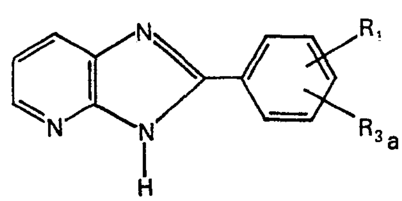

(IIa)

wherein
$R_1$ is defined as in claim 1 and
$R_{3a}$ represents an alkoxy group with 2 or 3 carbon atoms which is substituted by an aralkylthio group with 7 to 9 carbon atoms or by a phenylthio group optionally mono-, di- or tri-substituted by alkyl groups with 1 to 3 carbon atoms, alkoxy groups with 1 to 3 carbon atoms and/or halogen atoms, is oxidised and subsequently, if desired, a compound of general formula (I) thus obtained is converted into the physiologically acceptable acid addition salts thereof with inorganic or organic acids.
Priority: 11.07.1979 (P 29 27 987.6)

3. Process as claimed in claims 1 and 2, characterised in that the reaction is carried out in a solvent at temperatures of between −80 and 100°C.

4. Process as claimed in claims 1 to 3, characterised in that the oxidation is effected at ambient temperature, with one equivalent of the oxidising agent used.

5. Process as claimed in claims 1 to 4, characterised in that hydrogen peroxide is used as the oxidising agent.

**Revendications**

pour les Etats contractants BE, CH/LI, DE, FR, GB, IT, LU, NL, SE

1. 2-alcoxyphényl-imidazo[4,5-b]pyridines de formule générale:

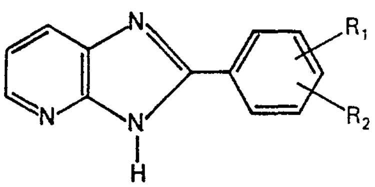
(I)

dans laquelle:
$R_1$ représente un groupe alcoxy avec 1 à 3 atomes de carbone et
$R_2$ représente un groupe alcoxy avec 2 ou 3 atomes de carbone substitué par un groupe aralcoylsulfinyle avec 7 à 9 atomes de carbone ou par un groupe phénylsulfinyle, le noyau phényle du groupe phénylsulfinyle pouvant être mono-, di- ou trisubstitué par un groupe nitro, par des groupes alcoyle avec 1 à 3 atomes de carbone, des groupes alcoxy avec 1 à 3 atomes de carbone et/ou des atomes d'halogène, leurs tautomères 3H et leurs sels d'addition physiologiquement supportables avec des acides minéraux ou organiques.

2. Composés de formule générale I selon la revendication 1, dans laquelle:
$R_1$ représente le groupe méthoxy et
$R_2$ représente un groupe éthoxy substitué en position 2 par un groupe benzylsulfinyle, méthylphénylsulfinyle, nitrophénylsulfinyle, méthoxyphénylsulfinyle, diméthoxyphénylsulfinyle ou diméthoxyméthylphénylsulfinyle, leurs tautomères 3H et leurs sels d'addition physiologiquement supportables avec des acides minéraux ou organiques.

3. Composés de formule générale I selon la revendication 1, dans laquelle:
$R_1$ et $R_2$ sont définis comme dans la revendication 2,
$R_1$ est fixé en position 4 et $R_2$ en position 2 du noyau phényle, leurs tautomères 3H et leurs sels d'addition physiologiquement supportables avec des acides minéraux ou organiques.

4. La 2-[2-(2-Phénylsulfinyl-éthoxy)-4-méthoxyphényl]-imidazo[4,5-b]pyridine et ses sels d'addition avec des acides.

5. La 2-[2-(2-(4-chlorophénylsulfinyl)-éthoxy)-4-méthoxyphényl]-imidazo[4,5-b]pyridine et ses sels d'addition avec des acides.

6. La 2-[2-(2-(4-méthylphénylsulfinyl)-éthoxy)-4-méthoxyphényl]-imidazo[4,5-bpyridine et ses sels d'addition avec des acides.

7. La 2-[2-(2-(2-méthyl-4,5-diméthoxyphénylsulfinyl) - éthoxy) - 4 - méthoxyphényl] - imidazo-[4,5-b]pyridine et ses sels d'addition avec des acides.

8. Utilisation des nouveaux composés selon les revendications 1–7 pour la préparation d'un médicament à activité hypertensive et/ou positivement inotrope par voie non chimique.

9. Médicament contenant un composé selon les revendications 1–7 conjointement à un ou plusieurs excipients et/ou diluants inertes.

10. Procédé pour la préparation des nouvelles 2-alcoxyphényl-imidazo[4,5-b]pyridines de formule générale selon la revendication 1:

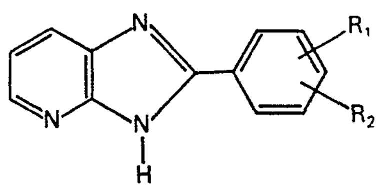
(I)

dans laquelle:
$R_1$ représente un groupe alcoxy avec 1 à 3 atomes de carbone et
$R_2$ représente un groupe alcoxy avec 2 ou 3 atomes de carbone substitué par un groupe aralcoylsulfinyle avec 7 à 9 atomes de carbone ou un groupe phénylsulfinyle, le noyau phényle du groupe phénylsulfinyle pouvant être mono-, di- ou trisubstitué par un groupe nitro, par des groupes alcoyle avec 1 à 3 atomes de carbone, des groupes alcoxy avec 1 à 3 atomes de carbone et/ou des atomes d'halogène, ainsi que de leurs tautomères 3H et de leurs sels d'addition physiologiquement supportables avec des acides minéraux ou organiques, caractérisé en ce qu'on oxyde une imidazo[4,5-b]pyridine de formule générale:

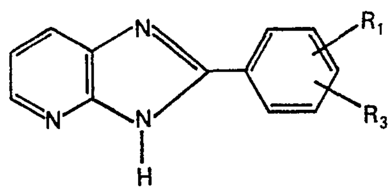
(II)

dans laquelle:
$R_1$ est défini comme au début et
$R_3$ représente un groupe alcoxy avec 2 ou 3 atomes de carbone, lequel est substitué par un groupe aralcoylthio avec 7 à 9 atomes de carbone ou par un groupe phénylthio éventuellement mono-, di- ou trisubstitué par un groupe nitro, par des groupes alcoyle avec 1 à 3 atomes de carbone, des groupes alcoxy avec 1 à 3 atomes de carbone et/ou des atomes d'halogène, et en ce qu'éventuellement on transforme ensuite un composé obtenu de formule générale I en ses sels physiologiquement supportables d'addition avec des acides minéraux ou organiques.

**Revendications**

pour l'Etat contractant: AT

1. Procédé pour la préparation des nouvelles 2-alcoxyphényl-imidazo[4,5-b]pyridines de formule générale:

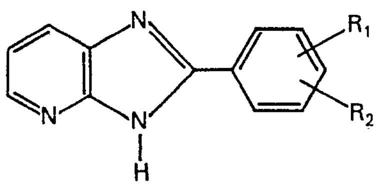
(I)

dans laquelle:
$R_1$ représente un groupe alcoxy avec 1 à 3 atomes de carbone et
$R_2$ représente un groupe alcoxy avec 2 ou 3 atomes de carbone substitué par un groupe aralcoylsulfinyle avec 7 à 9 atomes de carbone ou un groupe phénylsulfinyle, le noyau phényle du groupe phénylsulfinyle pouvant être mono-, di- ou trisubstitué par un groupe nitro, par des groupes alcoyle avec 1 à 3 atomes de carbone, des groupes alcoxy avec 1 à 3 atomes de carbone et/ou des atomes d'halogène, ainsi que de leurs tautomères 3H et de leurs sels d'addition physiologiquement supportables avec des acides minéraux ou organiques, caractérisé en ce qu'on oxyde une imidazo[4,5-b]pyridine de formule générale:

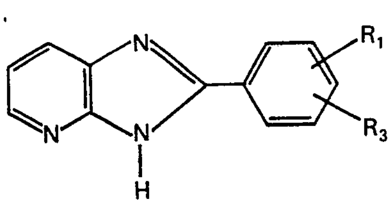
(II)

dans laquelle:
$R_1$ est défini comme au début et
$R_3$ représente un groupe alcoxy avec 2 ou 3 atomes de carbone, lequel est substitué par un groupe aralcoylthio avec 7 à 9 atomes de carbone ou par un groupe phénylthio éventuellement mono-, di- ou trisubstitué par un groupe nitro, par des groupes alcoyle avec 1 à 3 atomes de carbone, des groupes alcoxy avec 1 à 3 atomes de carbone et/ou des atomes d'halogène et on transforme éventuellement ensuite un composé de formule générale I obtenu en ses sels physiologiquement supportables d'addition avec des acides minéraux ou organiques.

2. Procédé selon la revendication 1 pour la préparation des novuelles 2-alcoxyphényl-imidazo[4,5-b]pyridines de formule générale:

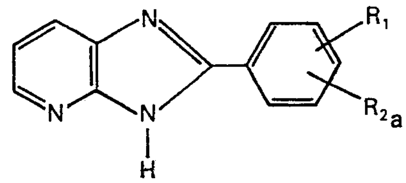
(Ia)

dans laquelle:
$R_1$ est défini comme dans la revendication 1 et
$R_{2a}$ représente un groupe alcoxy avec 2 ou 3 atomes de carbone substitué par un groupe aralcoylsulfinyle avec 7 à 9 atomes de carbone ou un groupe phénylsulfinyle, le noyau phényle du groupe phénylsulfinyle pouvant être mono-, di- ou trisubstitué par des groupes alcoyle avec 1 à 3 atomes de carbone, des groupes alcoxy avec 1 à 3 atomes de carbone et/ou des atomes d'halogène, ainsi que de leurs tautomères 3H et de leurs sels d'addition physiologiquement supportables avec des acides minéraux ou organiques, caractérisé en ce qu'on oxyde une imidazo[4,5-b]pyridine de formule générale:

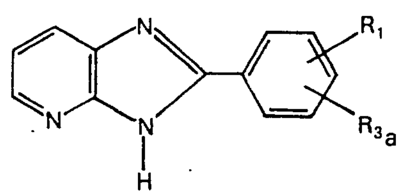
(IIa)

dans laquelle:
$R_1$ est défini comme dans la revendication 1 et
$R_{3a}$ représente un groupe alcoxy avec 2 ou 3 atomes de carbone, lequel est substitué par un groupe aralcoylthio avec 7 à 9 atomes de carbone ou par un groupe phénylthio éventuellement mono-, di- ou trisubstitué par des groupes alcoyle avec 1 à 3 atomes de carbone, des groupes alcoxy avec 1 à 3 atomes de carbone et/ou des atomes d'halogène, et on transforme éventuellement ensuite un composé de formule générale I obtenu en ses sels d'addition physiologiquement supportables avec des acides minéraux ou organiques.
Priorité: 11.07.1979 (P 29 27 987.6).

3. Procédé selon les revendications 1 et 2, caractérisé en ce que la réaction est effectuée dans un solvant à des températures entre -80 et 100°C.

4. Procédé selon les revendications 1 à 3, caractérisé en ce que l'oxydation est effectuée avec un équivalent de l'agent d'oxydation utilisé et à la température ambiante.

5. Procédé selon les revendications 1 à 4, caractérisé en ce qu'on utilise du peroxyde d'hydrogène en tant qu'agent d'oxydation.